(19) Europäisches Patentamt
European Patent Office
Office européen des brevets

(11) Publication number: **0 249 289 B1**

(12) **EUROPEAN PATENT SPECIFICATION**

(45) Date of publication of patent specification: **16.10.91**

(51) Int. Cl.⁵: **C07C 50/04**, C07C 50/12, C07C 46/06

(21) Application number: **87201075.6**

(22) Date of filing: **09.06.87**

(54) **Process for the preparation of quinones.**

(30) Priority: **11.06.86 IT 2075486**

(43) Date of publication of application:
**16.12.87 Bulletin 87/51**

(45) Publication of the grant of the patent:
**16.10.91 Bulletin 91/42**

(84) Designated Contracting States:
**AT BE CH DE ES FR GB GR LI LU NL SE**

(56) References cited:
**DE-A- 2 460 361**
**US-A- 3 297 445**

**CHEMICAL ABSTRACTS, vol. 84, 1976, page 452, abstract no. 43638r, Columbus, Ohio, US; & JP-A-75 95 231 (MITSUBISHI PETROCHEMICAL CO., LTD) 29-07-1975**

(73) Proprietor: **CONSIGLIO NAZIONALE DELLE RICERCHE**
**Viale Liegi 48/B**
**I-00198 Roma(IT)**

Proprietor: **ENICHEM SYNTHESIS S.p.A.**
**Via Ruggero Settimo 55**
**I-90139 Palermo(IT)**

(72) Inventor: **Minisci, Francesco**
**Via Marescalchi 19**
**I - 20133 Milan(IT)**
Inventor: **Citterio, Attilio**
**Piazzale Piola 5**
**I - 20131 Milan(IT)**
Inventor: **Vismara, Elena**
**Via G. Colombo 81/A**
**I - 20133 Milan(IT)**
Inventor: **De Bernardinis, Sivia**
**Via Forze Armate 253**
**I - 20152 Milan(IT)**
Inventor: **Neri, Carlo**
**Via Europa 32**
**I - 20097 San Donato Milanes Milan(IT)**

EP 0 249 289 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

Inventor: **Pallini Luciano**
**Via V. Bottego 3**
**I - 43045 Fornovo Taro Parma(IT)**
Inventor: **Correale Mariano**
**Via Volta 9**
**I - 24040 BonateSotto Bergamo(IT)**

(74) Representative: **Roggero, Sergio et al**
**Ing. Barzanò & Zanardo Milano S.p.A. Via Borgonuovo 10**
**I-20121 Milano(IT)**

## Description

The object of the present invention is a novel catalytic process for the synthesis of quinones. Such process is accomplished by oxidating 2,6-disubstituted phenols or diphenols with $H_2O_2$ in the presence of catalytic amounts of bromine, iodine, hydrogen bromide, or hydrogen iodide: the catalytic oxidation which is thus accomplished is extremely simple, cheap and highly selective.

Quinones are known to be an important class of compounds of the chemical industry; thus, 1,4-benzoquinone is used in the photographic industry and in the industry of the dyes; 1,4-naphthoquinone and 9.10-anthraquinone is used in the industry of dyes, 2-methylnaphthoquinone is a commercial Vitamin K, and 2,3,5-trimethyl-1,4-benzoquinone is an intermediate for the synthesis of vitamins, etc. (D.H. Barton "Comprehensive Organic Chemistry", Vol. 1, page 1,213, 1979). Furthermore, in as much as the quinones can be easily hydrogenated to yield diphenols, compounds which too are industrially useful, in particular in the photographic fields, and in the field of antioxidants, these latter may be conveniently obtained by starting from quinones, which are obtained, in their turn, by starting from mono-phenols, whenever these latter are easily accessible products.

A general method for the synthesis of quinones is constituted by the oxidation of phenols and diphenols with various oxidants, such as $CrO_3$, $Ag_2O$, $Ag_2CO_3$, $Ce^{IV}$ salts, the Fremy salt $(KO_3S)_2NO$, $(PhSeO)_2O$ and so forth. The use of these oxidants shows serious drawbacks from the industrial viewpoint, such as, e.g., not compatible costs (this is the case of the salts of Ag, Ce, of the Fremy salt, of selenium derivatives, etc.), or large amounts of salts to be disposed of (chrome salts), or highly toxic substances (selenium oxides).

The use of hydrogen peroxide, with different catalysts, in the oxidation of phenols, generally leads to the corresponding 1,2- and 1,4-diphenols (see, e.g., the German patent application No. 2,162,552, C.A. 86 189487d, C.A. 94 191302y and C.A. 85 139662d). A method is known as well for the oxidation of phenols to quinones, which uses hydrogen peroxide, but which is carried out in two steps, and provides first the synthesis of the analogous 4-chloro derivative, by means of the treatment of the phenol with HCl and $H_2O_2$ followed, in a second step, by the oxidation of this intermediate with $H_2O_2$ in acetic acid, to yield the quinone (see C.A. 85, 94070c).

The present Applicant has found now, and this is the object of the present patent application, that it is possible to catalytically oxidate, in an extremely simple, convenient and selective way, a 2,6-disubstituted phenol or diphenol with $H_2O_2$ in the presence of a catalyst selected from bromine, iodine, hydrogen bromide and hydrogen iodide, to yield the corresponding quinone. The reaction is generally applicable to 2,6-disubstituted mono-phenols, of both mono-, and di- and tri-cyclic structure, as well as to 1,2- and 1,4-diphenols. However, in view of the industrial interest of the end product, a preferred group of phenol starting products, which can be advantageously oxidated to the corresponding quinones, comprises those phenols of general formula (I):

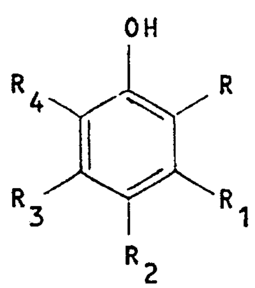

(I)

wherein R represents hydrogen, alkyl, alkoxy, aryl, halogen or hydroxy,
$R_1$ is hydrogen or alkyl,
$R_2$ can be hydrogen or hydroxy,
$R_3$ is hydrogen or alkyl,
$R_4$ represents hydrogen, alkyl, alkoxy, aryl, or halogen, or
R and $R_1$ and/or $R_3$ and $R_4$, when taken together, represent a butadienyl chain of formula

-CH = CH-CH = CH-

which forms a condensed ring on the phenol, with the proviso that R can be a hydrogen atom only when $R_2$ is hydroxy, and with the further proviso that $R_4$ can be hydrogen only when one of R and $R_2$ is hydroxy.

For the purposes of the present invention, the term "alkyl" means a straight or branched chain alkyl radical containing from 1 to 6 carbon atoms, such as, e.g., methyl, ethyl, propyl, isopropyl, butyl, sec.butyl, tert.butyl, pentyl, hexyl, etc. and, analogously, the term "alkoxy" identifies a straight or branched chain alkoxy radical containing from 1 to 6 carbon atoms, such as, e.g., methoxy, ethoxy, isopropoxy, tert.butoxy, pentyloxy etc.. The term "aryl" indicates a phenyl radical, or a phenyl radical substituted with one or more groups independently selected from alkyl, alkoxy and halogen. Finally, the term "halogen" indicates any of chlorine, bromine, iodine and fluorine.

The process according to the present invention consists essentially in oxidating the phenol with $H_2O_2$ in the presence of a catalyst selected from bromine, iodine, hydrogen bromide and hydrogen iodide. The reaction is preferably carried out by using commercial aqueous solutions of $H_2O_2$. Hence, when as the starting phenol one which is soluble in water is used, it is not necessary to use any other solvents, whilst, when the starting phenol is insoluble, or only partially soluble, in water, it is necessary to use an organic solvent which is miscible with water for making it possible the reaction to occur in homogeneous phase. Suitable solvents for the intended purpose are all of the water-miscible organic solvents which are capable of dissolving the phenol, and do not interfere negatively with the course of the oxidation reaction, such as, e.g., the lower alkanols, typically methanol and ethanol, the organic acids, e.g., acetic acid, acetonitrile, etc.. In the oxidation of the 2,6-disubstituted mono-phenols, the molar ratio between $H_2O_2$ and the phenol is preferably higher than 2, and still more preferably it is comprised within the range of from 2 to 3, whilst when diphenols are oxidated, such ratio is preferably comprised within the range of from 1 to 2, and still more preferably within the range of from 1 to 1.5. Higher molar ratios can be used, but they do not afford any further advantages for the same reaction. The reaction is carried out at a temperature generally comprised within the range of from 0$^\circ$C to the refluxing temperature of the reaction mixture and preferably, when mono-phenols are oxidated, at a temperature comprised within the range of from room temperature to the refluxing temperature of the reaction mixture, and, when the diphenols are oxidated, within the range of from 0$^\circ$C to 60$^\circ$C.

As to the catalyst, it was observed that, in the oxydation of the monophenols, a higher selectivity is obtained by using bromine or hydrogen bromide, and, still more preferably, bromine, rather than iodine or hydrogen iodide. In that case, very good results are obtained by using the catalyst, during the course of the reaction, at a molar concentration higher than 50% of the steady-state concentration of the mono-phenol. This situation can be accomplished both by reacting all of the reactants together and using a catalyst amount equal to at least 51% of the starting phenol, and, more conveniently, using a much lower catalyst amount, but adding, gradually over time, the phenol to the solution of the catalyst and hydrogen peroxide, in such a way that the steady-state concentration of the catalyst is always higher than 50% of the steady-state concentration of the phenol, due to the fact that the phenol reacts rapidly, and its concentration in the reaction medium remains always low, whilst the concentration of the catalyst remains substantially equal to its initial concentration.

In the oxidation of the diphenols, a better selectivity was observed on the contrary when iodine or hydrogen iodide, and still more preferably, iodine, is used as the catalyst. In this case, very good results were obtained by using catalyst amounts comprised within the range of from 0.1 to 10% by mol relatively to the diphenol used as the starting product.

If in the process of the present invention iodine and bromine are used as catalysts, the reaction rate can be increased, is desired, by initially adding a strong acid, which can be the same hydrogen bromide or hydrogen iodide, but which can also be sulphuric acid. This addition does not alter the very good selectivity of the process, but simply makes it faster. At the end of the oxidation reaction, the product is easily separated from the solution by crystallization, after possibly preliminarily removing a portion of the solvent. The process of the present invention leads to the corresponding quinone with high yields and with a high selectivity. This result appears so more surprising on considering that, under the same conditions, the other halogens or hydrogen halides do not yield any results, or give rise to extremely low yields and selectivities. Advantages of the process of oxidation of the phenols according to the present invention are the low cost of the oxidating agent and the fact that the reduction product (water) does not show disposal problems; the operative accomplishing thereof is furthermore extremely simple, which makes the methodology very versatile. The process of the present invention is disclosed in greater detail by the following examples, whose content must anyway not be understood as being limitative of the purview of the same invention.

Example 1

To a solution of 2,6-di-tert.butyl-phenol (11 g, 53.3 mmol), $H_2O_2$ at 59% (5 ml) and concentrated sulphuric acid (3 ml) in methanol (150 ml), bromine (1.5 ml) is added, and the so-obtained reaction mixture

is refluxed for 10 minutes. The solution is then concentrated to a small volume (50 ml) and the product is crystallized out by cooling, with 99%-pure (GLC) 2,6-di-tert.butyl-1,4-benzoquinone (10.8 g, 92%) being obtained.

Example 2

A solution of 2,6-di-tert.butyl-phenol (2.5 g, 12 mmol), concentrated sulphuric acid (3 ml), hydrogen peroxide at 60% (6 ml) and bromine (0.5 ml, 9.7 mmol) in methanol (50 ml) is refluxed, and to it a solution of 2,6-di-tert.butyl-phenol (8.5 g, 41.2 mmol) in methanol (100 ml) is added dropwise within a 40-minute time. On addition ended, the reaction mixture is refluxed for further 5 minutes, the solution is concentrated to small volume (50 ml) and 2,6-di-tert.butyl-1,4-benzoquinone (10.6 g, 90.4%) is crystallized.

Example 3

A solution of 2,6-di-tert.butyl-phenol (11 g, 53.3 mmol), hydrogen peroxide at 60% (8 ml) and hydrogen bromide (5 g, 61.7 mmol) in methanol (150 ml) is refluxed for 25 minutes. On concentrating the solution, 2,6-di-tert.butyl-1,4-benzoquinone (10.5 g, 89.4%) crystallizes. This product is hydrogenated, in methanol (110 ml), in autoclave, with Pd/C at 5% (0.12 g), under a hydrogen pressure of 10 $kg/cm^2$, for 50 minutes, at 20 °C. After the catalyst being filtered off, and solvent evaporation, 2,6-di-tert.butyl-hydroquinone is obtained with a yield of 97%.

Example 4

A solution of hydrogen peroxide at 60% (7 ml), 2,6-di-tert.butyl-phenol (2.5 g, 12 mmol) and hydrogen bromide (1 g, 12.3 mmol) in methanol (50 ml) is refluxed. Into it a solution of 2,6-di-tert.butyl-phenol (8.5 g, 41.2 mmol) in methanol (100 ml) is then added dropwise within a 40 minute time. The reaction mixture is heated for further 5 minutes, the solution is concentrated and 2,6-di-tert.butyl-1,4-benzoquinone (10.6 g, 90.4%) is crystallized.

Example 5

The process was carried out as in Example 1, but using 2,6-dimethyl-phenol instead of 2,6-di-tert.butyl-phenol, 2,6-dimethyl-1,4-benzoquinone being obtained with a yield of 85%.

Example 6

The process was carried out as in Example 1, but using 2,3,6-trimethyl-phenol instead of 2,6-di-tert.butyl-phenol, 2,3,6-trimethyl-1,4-benzoquinone being obtained with a yield of 77%.

Example 7

The process was carried out as in Example 1, but using 2-methyl-naphthol instead of 2,6-di-tert.butyl-phenol, 2-methyl-naphthoquinone being obtained with a yield of 94%.

Examples 8-17

General Methodology

A solution of a suitable diphenol (20 mmol) in methanol or acetic acid (60 ml) is treated 4 hours long with hydrogen peroxide at 60% (22 mmol), concentrated sulphuric acid (1 ml) and bromine or iodine or hydrogen iodide (1 mmol) at 25 °C.

2,6-Di-tert.butyl-1,4-benzoquinone and 1,4-naphthoquinone crystallize directly out from the solution, whilst in the other cases the quinone is isolated by evaporating the solvent. The results obtained are reported in following Table 1:

Table 1

| Example No. | Diphenol | Catalyst | Yield, % |
|---|---|---|---|
| 8 | Hydroquinone | $I_2$ | 97 |
| 9 | Hydroquinone | $Br_2$ | 52 |
| 10 | 2,3,5-trimethylhydroquinone | $I_2$ | 100 |
| 11 | 2,3,5-trimethylhydroquinone | $Br_2$ | 74 |
| 12 | 1,4-dioxynaphthalene | $I_2$ | 100 |
| 13 | 1,4-dioxynaphthalene | $Br_2$ | 72 |
| 14 | 2,6-di-tert.butyl-hydroquinone | $I_2$ | 86 |
| 15 | 2,6-di-tert.butyl-hydroquinone | $Br_2$ | 68 |
| 16 | 3,5-di-tert.butyl-pyrocatechol | $I_2$ | 76 |
| 17 | 3,5-di-tert.butyl-pyrocatechol | $Br_2$ | 48 |

Examples 18-19

General Methodology

A solution of diphenol (20 mmol) in methanol (60 ml) is treated with hydrogen iodide (2 mmol) and hydrogen peroxide at 60% (24 mmol) for 4 hours. By evaporating the solvent, the corresponding quinones are obtained. The results are shown in following Table 2:

Table 2

| Example No. | Diphenol | Catalyst | Yield, % |
|---|---|---|---|
| 18 | Hydroquinone | HI | 98 |
| 19 | 1,4-dioxy-naphthalene | HI | 96 |

Examples 20-23

General Methodology

13 mmol of hydroquinone (or of its methyl-derivative) is added to 40 ml of water; 17 mmol of $H_2O_2$ at 30% and 1.3 mmol of HI are added, and the whole mass is kept stirred for 4 hours at room temperature.

The corresponding quinones, which contain only traces of the diphenols used as the starting product, precipitate out.

Trimethyl-benzoquinone separates initially in an oily state, but solidifies after cooling.

The following yields are obtained:

| Example | Diphenol | Yield |
|---|---|---|
| 20 | hydroquinone | 94% |
| 21 | 2-methyl-hydroquinone | 95% |
| 22 | 2,6-dimethyl-hydroquinone | 92% |
| 23 | 2,3,4-trimethyl-hydroquinone | 88% |

## Claims

1. Process for the synthesis of quinones by means of the oxidation of 2,6-di-substituted mono-phenols or of diphenols, characterized in that the oxidation is carried out with $H_2O_2$ in the presence of catalytic amounts of an agent selected from bromine, iodine, hydrogen bromide and hydrogen iodide.

2. Process according to claim 1, wherein the phenol starting products are compounds having the general formula (I):

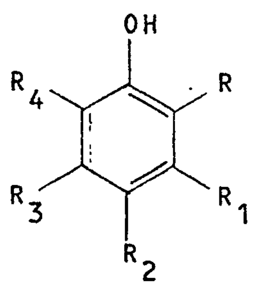

(I)

wherein R represent hydrogen, alkyl, alkoxy, aryl, halogen or hydroxy,
$R_1$ is hydrogen or alkyl,
$R_2$ can be hydrogen or hydroxy,
$R_3$ is hydrogen or alkyl,
$R_4$ represents hydrogen, alkyl, alkoxy, aryl, or halogen, or
R and $R_1$ and/or $R_3$ and $R_4$, when taken together, represent a butadienyl chain of formula

$-CH=CH-CH=CH-$

which forms a condensed ring on the phenol, with the proviso that R can be a hydrogen atom only when $R_2$ is hydroxy, and with the further proviso that $R_4$ can be hydrogen only when one of R and $R_2$ is hydroxy.

3. Process according to claim 1, wherein $H_2O_2$ in aqueous solution is used.

4. Process according to claim 3, wherein the reaction is carried out in the presence of a solvent capable of dissolving the phenol starting product, which is miscible with water and does not interfere negatively with the course of the reaction.

5. Process according to claim 4, wherein such solvent is selected from the lower alkanols and the organic acids.

6. Process according to claim 5, wherein such solvent is selected from methanol, ethanol and acetic acid.

7. Process according to claim 1, wherein the reaction is carried out at a temperature comprised within the range of from 0° C to the refluxing temperature of the reaction mixture.

8. Process according to claim 1, wherein the phenol starting product is a 2,6-di-substituted mono-phenol.

9. Process according to claim 8, wherein the catalyst is selected from bromine and hydrogen bromide.

10. Process according to claim 9 wherein the catalyst is bromine.

11. Process according to claim 8, wherein hydrogen peroxide is used in such an amount that the molar ratio of hydrogen peroxide to the phenol starting product is higher than 2.

12. Process according to claim 11, wherein such ratio is comprised within the range of from 2 to 3.

13. Process according to claim 8, wherein during the course of the reaction the molar concentration of the catalyst is higher than 50% of the steady-state concentration of the phenol.

14. Process according to claim 8, wherein the temperature is comprised within the range of from room temperature to the refluxing temperature of the reaction mixture.

15. Process according to claim 1, wherein the phenol starting product is a diphenol.

16. Process according to claim 15, wherein the catalyst is selected from iodine and hydrogen iodide.

17. Process according to claim 16, wherein the catalyst is iodine.

18. Process according to claim 15, wherein hydrogen peroxide is used in such an amount that the molar ratio of hydrogen peroxide to the phenol starting product is comprised within the range of from 1 to 2.

19. Process according to claim 18, wherein said ratio is comprised within the range of from 1 to 1.5.

20. Process according to claim 15, wherein the catalyst is used in an amount comprised within the range of from 0.1 to 10% by mol relatively to the diphenol used as the starting product.

21. Process according to claim 15, wherein the temperature is comprised within the range of from 0 ° C to 60 ° C.

22. Process according to claim 1, wherein the reaction is carried out in the presence of a strong acid.

23. Process according to claim 22, wherein such strong acid is sulphuric acid.

**Revendications**

1. Procédé de synthèse de quinones par oxydation de monophénols 2,6-disubstitués ou de diphénols, caractérisé en ce que l'oxydation est effectuée à l'aide d'$H_2O_2$, en présence de quantités catalytiques d'un agent choisi parmi le brome, l'iode, le bromure d'hydrogène et l'iodure d'hydrogène.

2. Procédé conforme à la revendication 1, dans lequel les phénols de départ sont des composés présentant la formule générale (I) :

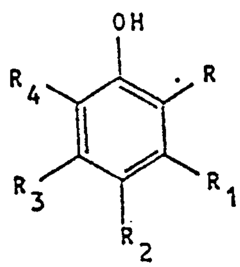

(I)

dans laquelle R représente un atome d'hydrogène ou d'halogène ou un groupe alkyle, alcoxy, aryle ou hydroxy,
$R_1$ représente un atome d'hydrogène ou un groupe alkyle,
$R_2$ peut représenter un atome d'hydrogène ou un groupe hydroxy,
$R_3$ représente un atome d'hydrogène ou un groupe alkyle,
$R_4$ représente un atome d'hydrogène ou d'halogène ou un groupe alkyle, alcoxy ou aryle, ou bien
R et $R_1$ et/ou $R_3$ et $R_4$, pris conjointement, représentent un chainon butadiényle de formule

-CH = CH-CH = CH-

qui forme un cycle condensé sur le phénol,
avec la condition que R ne peut représenter un atome d'hydrogène que quand $R_2$ représente un groupe hydroxy, et avec la condition supplémentaire que $R_4$ ne peut représenter un atome d'hydrogène que quand l'un de R et $R_2$ représente un groupe hydroxy.

**3.** Procédé conforme à la revendication 1, dans lequel on utilise $H_2O_2$ à l'état de solution aqueuse.

**4.** Procédé conforme à la revendication 3, dans lequel on effectue la réaction en présence d'un solvant qui est capable de dissoudre le phénol de départ, qui est miscible à l'eau et qui n'influence pas de façon négative le déroulement de la réaction.

**5.** Procédé conforme à la revendication 4, dans lequel on choisit un tel solvant parmi les alcanols inférieurs et les acides organiques.

**6.** Procédé conforme à la revendication 5, dans lequel on choisit le solvant parmi le méthanol, l'éthanol et l'acide acétique.

**7.** Procédé conforme à la revendication 1, dans lequel on effectue la réaction à une température située dans l'intervalle allant de 0 °C à la température de reflux du mélange réactionnel.

**8.** Procédé conforme à la revendication 1, dans lequel le phénol de départ est un monophénol 2,6-disubstitué.

**9.** Procédé conforme à la revendication 8, dans lequel le catalyseur est choisi parmi le brome et le bromure d'hydrogène.

**10.** Procédé conforme à la revendication 9, dans lequel le catalyseur est du brome.

**11.** Procédé conforme à la revendication 8, dans lequel on utilise le peroxyde d'hydrogène en une quantité telle que le rapport molaire du peroxyde d'hydrogène au phénol de départ a une valeur supérieure à 2.

**12.** Procédé conforme à la revendication 11, dans lequel la valeur dudit rapport se situe dans l'intervalle allant de 2 à 3.

**13.** Procédé conforme à la revendication 8, dans lequel, au cours du déroulement de la réaction, la concentration molaire du catalyseur est supérieure à 50% de la concentration instantanée du phénol.

**14.** Procédé conforme à la revendication 8, dans lequel la température est située dans l'intervalle allant de la température ambiante à la température de reflux du mélange réactionnel.

**15.** Procédé conforme à la revendication 1, dans lequel le phénol de départ est un diphénol.

**16.** Procédé conforme à la revendication 15, dans lequel le catalyseur est choisi parmi l'iode et l'iodure d'hydrogène.

**17.** Procédé conforme à la revendication 16, dans lequel le catalyseur est de l'iode.

**18.** Procédé conforme à la revendication 15, dans lequel on utilise le peroxyde d'hydrogène en une

quantité telle que le rapport molaire du peroxyde d'hydrogène au phénol de départ a une valeur comprise dans l'intervalle allant de 1 à 2.

**19.** Procédé conforme à la revendication 18, dans lequel ledit rapport a une valeur comprise dans l'intervalle allant de 1 à 1,5.

**20.** Procédé conforme à la revendication 15, dans lequel on utilise le catalyseur en une quantité comprise dans l'intervalle allant de 0,1 à 10% en moles, par rapport au diphénol utilisé comme produit de départ.

**21.** Procédé conforme à la revendication 15, dans lequel la température se situe dans l'intervalle allant de 0 °C à 60 °C.

**22.** Procédé conforme à la revendication 1, dans lequel la réaction est effectuée en présence d'un acide fort.

**23.** Procédé conforme à la revendication 22, dans lequel ledit acide fort est l'acide sulfurique.

**Patentansprüche**

**1.** Verfahren zur Synthese von Chinonen durch Oxidation von 2,6-disubstituierten Monophenolen oder von Diphenolen, dadurch gekennzeichnet, daß die Oxidation mit Wasserstoffperoxid in Gegenwart katalytischer Mengen eines unter Brom, Iod, Bromwasserstoff und Iodwasserstoff ausgewählten Mittels ausgeführt wird.

**2.** Verfahren nach Anspruch 1, worin die Phenol-Ausgangsprodukte Verbindungen mit der allgemeinen Formel (I):

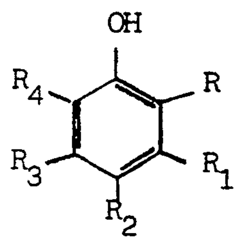

(I)

sind, worin

| | |
|---|---|
| R | für Wasserstoff, Alkyl, Alkoxy, Aryl, Halogen oder Hydroxy steht, |
| $R_1$ | Wasserstoff oder Alkyl bedeutet, |
| $R_2$ | Wasserstoff oder Hydroxy sein kann, |
| $R_3$ | Wasserstoff oder Alkyl ist, |
| $R_4$ | für Wasserstoff, Alkyl, Alkoxy, Aryl oder Halogen steht oder |
| R und $R_1$ und/oder $R_3$ und $R_4$, | zusammengenommen, eine Butadienylkette der Formel<br>-CH = CH-CH = CH-<br>bedeuten, die an dem Phenol einen kondensierten Ring ausbildet, mit der Maßgabe, daß R nur dann ein Wasserstoffatom bedeuten kann, wenn $R_2$ Hydroxy darstellt, und mit der weiteren Maßgabe, daß $R_4$ nur dann Wasserstoff bedeuten kann, wenn einer der Reste von R und $R_2$ für Hydroxysteht. |

**3.** Verfahren nach Anspruch 1, worin Wasserstoffperoxid in wäßriger Lösung verwendet wird.

**4.** Verfahren nach Anspruch 3, worin die Umsetzung in Gegenwart eines zum Auflösen des Phenolausgangsproduktes befähigten Lösungsmittels ausgeführt wird, das mit Wasser mischbar ist und den Ablauf der Reaktion nicht nachteilig beeinflußt.

5. Verfahren nach Anspruch 4, worin das Lösungsmittel unter niederen Alkanolen und organischen Säuren ausgewählt wird.

6. Verfahren nach Anspruch 5, worin das Lösungsmittel unter Methanol, Ethanol und Essigsäure ausgewählt wird.

7. Verfahren nach Anspruch 1, worin die Umsetzung bei einer Temperatur im Bereich von 0°C bis zur Rückflußtemperatur des Reaktionsgemisches ausgeführt wird.

8. Verfahren nach Anspruch 1, worin das Phenolausgangsprodukt ein 2,6-disubstituiertes Monophenol ist.

9. Verfahren nach Anspruch 8, worin der Katalysator unter Brom und Bromwasserstoff ausgewählt wird.

10. Verfahren nach Anspruch 9, worin der Katalysator Brom ist.

11. Verfahren nach Anspruch 8, worin das Wasserstoffperoxid in einer solchen Menge verwendet wird, daß das Molverhältnis von Wasserstoffperoxid zu Phenolausgangsprodukt größer als 2 ist.

12. Verfahren nach Anspruch 11, worin das Verhältnis im Bereich von 2 bis 3 liegt.

13. Verfahren nach Anspruch 8, worin während des Reaktionsablaufes die Molkonzentration des Katalysators höher als 50% der Gleichgewichtskkonzentration des Phenols liegt.

14. Verfahren nach Anspruch 8, worin die Temperatur im Bereich von Raumtemperatur bis zur Rückflußtemperatur des Reaktionsgemisches liegt.

15. Verfahren nach Anspruch 1, worin das Phenolausgangsprodukt ein Diphenol ist.

16. Verfahren nach Anspruch 15, worin der Katalysator unter Iod und Iodwasserstoff ausgewählt wird.

17. Verfahren nach Anspruch 16, worin der Katalysator Iod ist.

18. Verfahren nach Anspruch 15, worin das Wasserstoffperoxid in einer solchen Menge eingesetzt wird, daß das Molverhältnis von Wasserstoffperoxid zu dem Phenolausgangsprodukt im Bereich von 1 bis 2 liegt.

19. Verfahren nach Anspruch 18, worin das Verhältnis im Bereich 1 bis 1,5 liegt.

20. Verfahren nach Anspruch 15, worin der Katalysator in einer Menge im Bereich von 0,1 bis 10 Mol-%, bezogen auf das als Ausgangsprodukt verwendete Diphenol, eingesetzt wird.

21. Verfahren nach Anspruch 15, worin die Temperatur im Bereich von 0°C bis 60°C gehalten wird.

22. Verfahren nach Anspruch 1, worin die Umsetzung in Gegenwart einer starken Säure ausgeführt wird.

23. Verfahren nach Anspruch 22, worin die starke Säure Schwefelsäure ist.